(19) Europäisches Patentamt
European Patent Office
Office européen des brevets

(11) Publication number : 0 538 001 A1

(12) # EUROPEAN PATENT APPLICATION

(21) Application number : 92309350.4

(51) Int. Cl.$^5$ : **C07D 477/00**, A61K 31/40

(22) Date of filing : 14.10.92

(30) Priority : 17.10.91 US 777978

(43) Date of publication of application : 21.04.93 Bulletin 93/16

(84) Designated Contracting States : CH DE FR GB IT LI NL

(71) Applicant : MERCK & CO. INC.
126, East Lincoln Avenue P.O. Box 2000
Rahway New Jersey 07065-0900 (US)

(72) Inventor : Dininno, Frank
5 Benjamin Court
Old Bridge, NJ 08857 (US)
Inventor : Guthikonda, Ravindra N.
289 Savoy Avenue
Edison, NJ 08820 (US)

(74) Representative : Thompson, John Dr. et al
Merck & Co., Inc. European Patent
Department Terlings Park Eastwick Road
Harlow, Essex CM20 2QR (GB)

(54) **MRSA active 2-halophenyl carbapenems.**

(57) Carbapenems of the formula

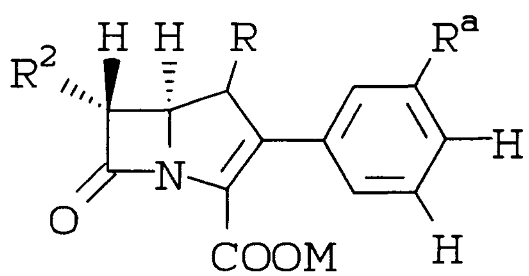

where $R^a$ is Br or I, are useful antibacterial agents.



Jouve, 18, rue Saint-Denis, 75001 PARIS

## BACKGROUND OF THE INVENTION

The present invention relates to antibacterial agents of the carbapenem class, in which the 2-position side-chain is characterized by a 2-phenyl moiety, substituted by various substituents, as described in more detail further below.

Thienamycin was an early carbapenem antibacterial agent having a broad spectrum; it has the following formula:

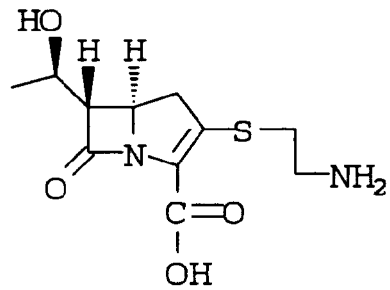

Later, N-formimidoyl thienamycin was discovered; it has the formula:

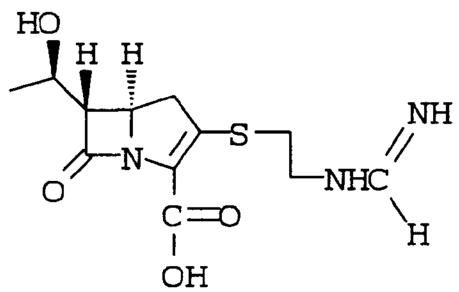

The 2-phenyl-carbapenems of the present invention are not necessarily of interest for a broad antibacterial spectrum such as that of thienamycin or N-formimidoyl thienamycin. Rather, their spectrum of activity of primary interest is to gram positive microorganisms, especially methicillin resistant Staphylococcus aureus (MRSA), methicillin resistant Staphylococcus epidermidis (MRSE), and methicillin resistant coagulase negative Staphylococci (MRCNS). The antibacterial compounds of the present invention thus comprise an important contribution to therapy of these difficult to control pathogens. Moreover, there is an increasing need for agents effective against such pathogens (MRSA/MRCNS) which are at the same time safe, i.e., free from undesirable toxic side effects. No β-lactam antibacterial has yet been found which meets these requirements. And, the current agent of choice, vancomycin, a glycopeptide antibacterial, is experiencing an ever increasing amount of resistance in the MRSA/MRCNS pathogens.

More recently, carbapenem antibacterial agents have been described which have a 2-substituent which is an aryl moiety optionally substituted by, e.g., aminomethyl and substituted aminomethyl. These agents are described in U.S. Patent Nos. 4,543,257 and 4,260,627 and have the formula:

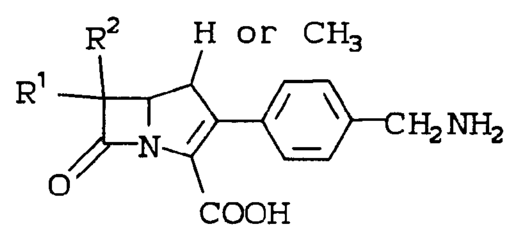

However, there is no description or suggestion of the suprisingly better anti-MRSA/MRCNS activity of the compounds of the present invention.

U.S. Pat. No. 4,978,659 describes a particular class of compounds of the formula:

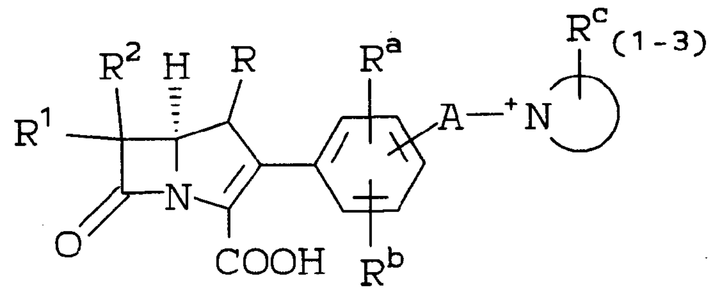

but this limited teaching in no way suggests the surprisingly better anti-MRSA/MRCNS activity of the compounds of the instant invention.

Therefore there is provided by the present invention a group of meta-substituted 2-halophenyl carbapenems that have unexpected levels of anti-MRSA/MRCNS activity. Critically, Applicants have found this unexpected level of activity to depend upon the particular halogen and upon meta-phenyl substitution.

SUMMARY OF INVENTION

The present invention provides novel carbapenem compounds of the formula:

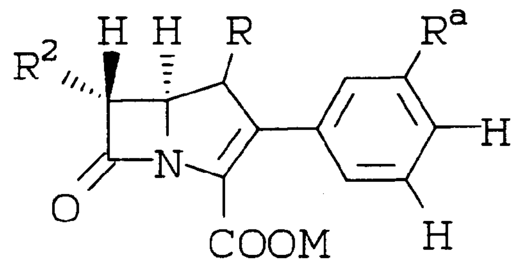

I

wherein R is H or $CH_3$, $R^2$ is (R)-$CH_3CH(OH)$- or (R)-$CH_3CH(F)$-, M is a pharmaceutically acceptable cation or ester and $R^a$ is I or Br.

DETAILED DESCRIPTION OF THE INVENTION

It has been found in certain carbapenems that with certain 2-side-chain selections, the ultimate balance of properties in the overall molecule may be enhanced by selection of a 6-(R)(1-fluoroethyl) moiety instead of a 6-(R)(1-hydroxyethyl). Preparation of 6-fluoroalkyl compounds within the scope of the present invention is carried out in a straightforward manner using techniques well known in the art of preparing carbapenem antibacterial compounds. See, e.g., J. G. deVries et al., Heterocycles, 1985, 23, 1915; BE 900 718 A (Sandoz) and Japanese Patent Pub. No. 6-0163-882-A (Sanruku Ocean).

In preferred compounds of Formula I $R^1$ is hydrogen and $R^2$ is 6-(R)-(1-hydroxyethyl). While R = H is usually preferred, there are instances in which R = $CH_3$ might provide improved chemical stability, water solubility, or pharmacokinetic behavior. The substituent R = $CH_3$ may be of either configuration, i.e., the $\alpha$ or $\beta$-stereoisomer.

The carbapenem compounds of the present invention are useful per se and in their pharmaceutically acceptable salt and ester forms in the treatment of bacterial infections in animal and human subjects. The term "pharmaceutically acceptable ester or salt" refers to those salt and ester forms of the compounds of the present invention which would be apparent to the pharmaceutical chemist, i.e. , those which are non-toxic and which would favorably affect the pharmacokinetic properties of said compounds, their palatability, absorption, distribution, metabolism and excretion. Other factors, more practical in nature, which are also important in the selection, are cost of the raw materials, ease of crystallization, yield, stability, hygroscopicity, and flowability of the resulting bulk drug. Conveniently, pharmaceutical compositions may be prepared from the active ingredients in combination with pharmaceutically acceptable carriers. Thus, the present invention is also concerned with pharmaceutical compositions and methods of treating bacterial infections utilizing as an active ingredient the novel carbapenem compounds of the present invention.

The pharmaceutically acceptable salts referred to above may take the form -COOM. The M may be an alkali metal cation such as sodium or potassium. Other pharmaceutically acceptable cations for M may be calcium, magnesium, zinc, ammonium, or alkylammonium cations such as tetramethylammonium, tetrabutylammonium, choline, triethylhydroammonium, meglumine, triethanolhydroammonium, etc.

The pharmaceutically acceptable esters of the novel carbapenem compounds of the present invention are such as would be readily apparent to a medicinal chemist, and include, for example, those described in detail in U.S. Pat. No. 4,309,438, Column 9, line 61 to Column 12, line 51, which is incorporated herein by reference. Included within such pharmaceutically acceptable esters are those which are hydrolyzed under physiological conditions, such as pivaloyloxymethyl, acetoxymethyl, phthalidyl, indanyl and methoxymethyl, and those described in detail in U.S. Pat. No. 4,479,947, which is incorporated herein by reference.

The compounds of the present invention are valuable antibacterial agents active against various Gram-positive and to a lesser extent Gram-negative bacteria and accordingly find utility in human and veterinary medicine. The antibacterials of the invention are not limited to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The compounds of this invention may be used in any of a variety of pharmaceutical preparations. They may be employed in capsule, powder form, in liquid solution, or in suspension. They may be administered by a variety of means; those of principal interest include: topically or parenterally by injection (intravenously or intramuscularly).

Compositions for injection, a preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a suitable vehicle, such as sterile water. Topical applications may be formulated in hydrophobic or hydrophilic bases as ointments, creams, lotions, paints, or powders.

The dosage to be administered depends to a large extent upon the condition and size of the subject being treated as well as the route and frequency of administration, the parenteral route by injection being preferred for generalized infections. Such matters, however, are left to the routine discretion of the therapist according to principles of treatment well known in the antibacterial art. Another factor influencing the precise dosage regimen, apart from the nature of the infection and peculiar identity of the individual being treated, is the molecular weight of the chosen species of this invention.

The compositions for human delivery per unit dosage, whether liquid or solid, may contain from 0.1% to 99% of active material, the preferred range being from about 10-60%. The composition will generally contain from about 15 mg to about 1500 mg of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution.

The preferred method of administration of the Formula I antibacterial compounds is parenteral by i.v. infusion, i.v. bolus, or i.m. injection.

For adults, 5-50 mg of Formula I antibacterial compounds per kg of body weight given 2, 3, or 4 times per day is preferred. Preferred dosage is 250 mg to 1000 mg of the Formula I antibacterial given two (b.i.d.) three (t.i.d.) or four (q.i.d.) times per day. More specifically, for mild infections a dose of 250 mg t.i.d. or q.i.d. is recommended. For moderate infections against highly susceptible gram positive organisms a dose of 500 mg t.i.d. or q.i.d. is recommended. For severe, life-threatening infections against organisms at the upper limits of sensitivity to the antibiotic, a dose of 1000 mg t.i.d. or q.i.d. is recommended.

For children, a dose of 5-25 mg/kg of body weight given 2, 3, or 4 times per day is preferred; a dose of 10 mg/kg t.i.d. or q.i.d. is usually recommended.

Antibacterial compounds of Formula I are of the broad class known as carbapenems or 1-carbade-thiapenems. Naturally occuring carbapenems are susceptible to attack by a renal enzyme known as dehydropeptidase (DHP). This attack or degradation may reduce the efficacy of the carbapenem antibacterial agent. The compounds of the present invention, on the other hand, are significantly less subject to such attack, and therefore may not require the use of a DHP inhibitor. However, such use is optional and contemplated to be part of the present invention. Inhibitors of DHP and their use with carbapenem antibacterial agents are disclosed in the prior art [see European Patent Applications No. 79102616.4 filed July 24, 1979 (Patent No. 0 007 614); and No. 82107174.3, filed August 9, 1982 (Publication No. 0 072 014)].

The compounds of the present invention may, where DHP inhibition is desired or necessary, be combined or used with the appropriate DHP inhibitor as described in the aforesaid patents and published application. Thus, to the extent that the cited European patent applications 1.) define the procedure for determining DHP susceptibility of the present carbapenems and 2.) disclose suitable inhibitors, combination compositions and methods of treatment, they are incorporated herein by reference. A preferred weight ratio of Formula I compound: DHP inhibitor in the combination compositions is about 1:1. A preferred DHP inhibitor is 7-(L-2-amino-2-carboxyethylthio)-2-(2,2-dimethylcyclopropanecarboxamide)-2-heptenoic acid or a useful salt thereof.

EXAMPLE 1

Step (a)

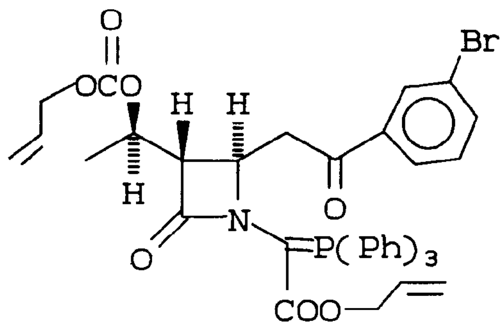

Preparation of (3S,4R)-1-[[(allyloxy)carbonyl)](triphenylphosphoranylidene)methyl]-3-[(1R)-1-[allyloxycarbonyloxy)ethyl]-4-[[[(3-bromo)phenyl]carbonyl]methyl]azetidin-2-one:

Magnesium (264mG,11mM) was added to a solution of 1,3-dibromobenzene (2.36G,10mM) in 20mL of anhydrous tetrahydrofuran. ~8μL of 1,2-dibromo-ethane was added. After 2 hr. stirring at room temperature under nitrogen, most of the metal was digested and the resulting solution was used as 0.5 molar solution of 3-bromophenylmagnesiumbromide.

This Grignard solution (8mL) was added dropwise to a solution of 1.4G (2mM) of (3S,4R)-1-[[(allyloxy)carbonyl](triphenylphosphoranylidene)methyl]-3-[(1R)-1-[[(allyloxy)carbonyloxy]ethyl]-4-[(1R)-2′-[(pyridylthio)c arbonyl]methyl]azetidin-2-one in 5 mL of tetrahydrofuran at 0°C under nitrogen. After 15 minutes, 10mL of saturated ammonium chloride solution was added, diluted with 25mL of ethyl acetate and washed with 3 x 10mL of saturated sodium chloride solution. After drying over anhydrous magnesium sulfate, solvent was removed from the organic phase to give a crude oil (yellow), which was chromatographed on silica gel using 2:3 mixture of ethyl acetate:hexane to give 720mG of the desired ylide ketone as cream colored foam.

Step (b)

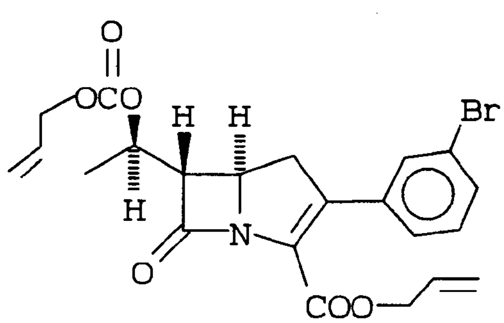

Preparation of allyl (5R,6S)-2-[3-bromophenyl]-6-[(1R)-allyloxycarbonyloxyethyl]-carbapen-2-em-3-carboxylate:

A solution of the ylid ketone (650mG), from step (a) above, and two tiny crystals of hydroquinone in 4mL of p-xylene was heated 3 hours at 130° under nitrogen. After cooling the reaction mixture, it was applied on a

silica gel column packed with hexane. The column was eluted first with hexane and then with 1:3 mixture of ethyl acetate:hexane to give 300mG of the desired carbapenem as colorless oil.

Step (c)

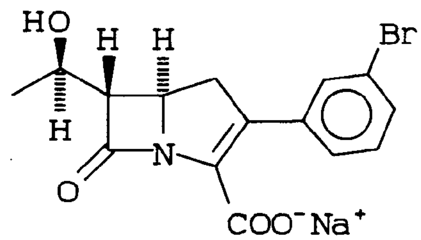

Preparation of sodium (5R,6S)-2-[3-bromophenyl]-6[(1R)-1-hydroxyethyl]-carbapen-2-em-3-carboxylate:

To a stirred solution of bis-allyl carbapenem of step (b), (225mG,0.4727mM) in 5mL of 1:1 mixture of $CH_2Cl_2$:ether in a centrifuge tube under $N_2$ at 0° were added 2-ethylhexanoic acid (75µL,0.472mM), triphenylphosphine (33mG, 0.135mM), tetrakis-triphenylphosphine palladium (84mG,0.075 mM), and sodium 2-ethylhexanoate (78mG, 0.472mM) sequentially. After 5 mins., a voluminous precipitate was observed. After an additional stirring of 2 hours, the reaction mixture was diluted with 10mL of ether. After centrifugation, liquid was decanted. The solid was washed with 2mL of ethylacetate. The resulting solid was dissolved in 2mL of water and applied on 2 x 1000µ reverse phase silica gel plates, and eluted with 1:4 mixture of acetonitrile and water. U.V. active area was scraped and stirred with 10mL of 4:1 $CH_3CN$:water mixture. Solid was filtered and washed with 2x4mL of the same solvent. The filtrate was washed with 4 x 25mL of hexane, and concentrated to ~2mL and freeze dried to give 104mG of the desired sodium salt as a white fluffy mass.

EXAMPLE 2

step (a)

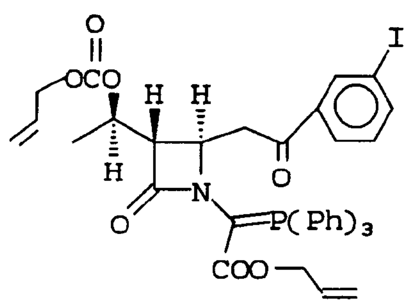

Preparation of (3S,4R)-1-[[(allyloxy)carbonyl)](triphenylphosphoranylidene)methyl]-3-[(1R)-1-(allyloxycarbonyloxy)ethyl]-4-[[[(3,5-diiodo)phenyl]carbonyl]methyl]azetidin-2-one:

A n-butyllithium solution (2.5M;2.1mL;5.25mM) was added dropwise to a stirred suspension of m-diiodobenzene (1.65G;5mM) in 10 ml of dry tetrahydrofuran at -78°C under nitrogen. After stirring 10 mins., a solution of magnesium bromide, prepared from 264 mG (11mM) of magnesium and 872 µL (10 mM) of 1,2-dibromoethane in 40 mL of anhydrous tetrahydrofuran, was added dropwise over 10 mins. The reaction mixture was stirred for 15 mins at -78°C and 30 mins. at 0°C. The resulting solution was used as 0.1 molar solution of m-iodophenyl magnesium bromide.

This solution was slowly added to a solution of 704 mG (1 mM) of the pyridylthioester as described in step (a) of Example 1 to give 400 mG of the desired ylid ketone as light yellow foam.

step (b)

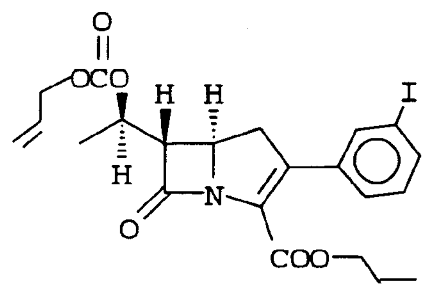

Preparation of allyl (5R,6S)-2-[(3,5-diiodo)phenyl]-6-[1R-(allyloxy)carbonyloxyethyl]-carbapen-2-em-3-carboxylate:

This carbapenem was prepared from the ylid ketone of step (a) of this example, using the procedure of step (b) of Example 1.

step (c)

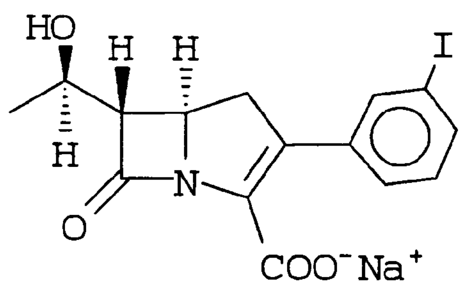

Preparation of sodium (5R,6S)-2-[(3,5-diiodo)phenyl]-6-[1R-1-hydroxyethyl]-carbapen-2-em-3-carboxylate:

This sodium salt was prepared from the bis-allyl protected carbapenem of step (b) of this example, using the procedure of step (c) of Example 1.

COMPARATIVE EXAMPLES

The comparative compounds of Table VII were produced analogously to the compounds of Examples 1 and 2 and the characterizing data is reported in Tables I to VI. Specifically, the comparative 2-fluorophenyl, 2-chlorophenyl and 2-bromophenyl compounds were produced analogously to example 1 from the appropriate Grignard Reagent. Likewise the 2-iodophenyl compounds were produced analogously to Example 2 from a diiodo compound.

CHARACTERIZING DATA

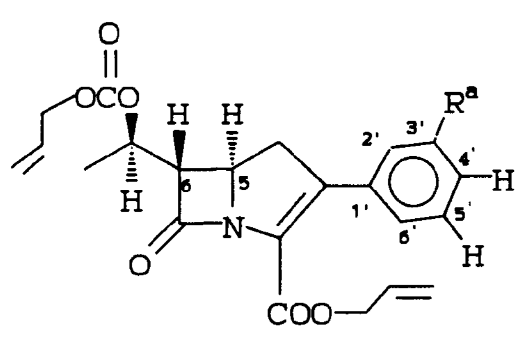

TABLE I

| $R^a$ | IR($cm^{-1}$) (C=O) B-LACTAM | CARBONATE | ESTER |
|---|---|---|---|
| F | 1780 | 1740 | 1720 |
| Cl | 1785 | 1745 | 1720 |
| Br | 1780 | 1740 | 1720 |
| I | 1780 | 1740 | 1720 |

TABLE II

| NMR | | | |
|---|---|---|---|
| $R^a$ | $H_5$ | H6 | $H_{2'}$ - $H_{6'}$ |
| F | 4.22-4.37 ddd;J-3,9 | 3.38-3.47 dd; J-3&3.5Hz | 7-7.38 AROMATIC H's |
| Cl | 4.22-4.37 ddd;J-3,8.5 &9Hz | 3.38-3.47 dd;J-3.&8Hz | 7.2-7.36 AROMATIC H's |
| Br | 4.22-4.36 ddd;J-3.9 9.5Hz | 3.38-3.46 dd;J-3&8.5Hz | 7.16-4.5 AROMATIC H's |
| I | 4.22-4.35 ddd; J-3.9&8Hz | 3.38-3.46 dd;J-3&8Hz | 7.03-7.7 AROMATIC H's |

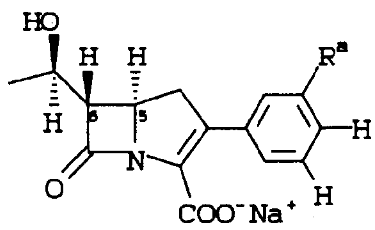

TABLE III

| UV(nm) | | | |
|---|---|---|---|
| $R^{al}$ | $\lambda_{max}$ | $\varepsilon_{ext}$ | NMR($D_20$) |
| F | ~308 | 7356 | 3.38-3.46(H6;dd;J=3&6H 6.94-7.33 (AROMATIC H's) |
| Cl | ~300 | 6815 | 3.38-3.46(H6;dd;J=3&6H 7.17-7.35 (AROMATIC H's) |
| Br | ~300 | 7738 | 3.43-3.50(H6;dd;J=3&6H 7.18-7.52 (AROMATIC H's) |
| I | ~302 | 7814 | 3.38-3.46(H6;dd;J=3&6H 6.97-7.66 (AROMATIC H's) |

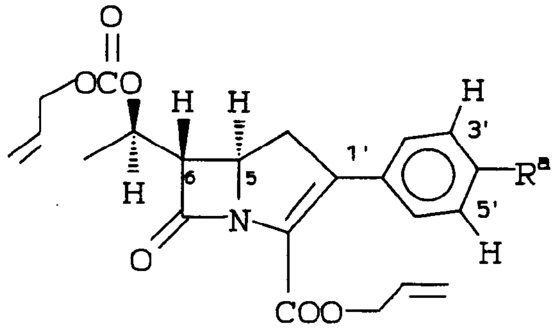

**TABLE IV**

**IR($cm^{-1}$)**

| | (C=O) | | |
|---|---|---|---|
| $R^a$ | B-LACTAM | CARBONATE | ESTER |
| F | 1780 | 1745 | 1720 |
| Cl | 1785 | 1745 | 1725 |
| Br | 1785 | 1745 | 1720 |
| I | 1780 | 1745 | 1720 |

TABLE V

| NMR | | | |
|---|---|---|---|
| $R^a$ | $H_5$ | $H_6$ | $H_{2'}$-$H_{6'}$ |
| F | 4.21-4.33 ddd;J=3, 9 &9.5Hz | 3.37-3.45 dd:J=3&8.5Hz | 6.98-7.41 (AROMATIC H's) |
| Br | 4.22-4.34 ddd;J=3, 9.5 &9Hz | 3.38-3.46 dd:J=3&8Hz | 7.2-7.52 (AROMATIC H's) |

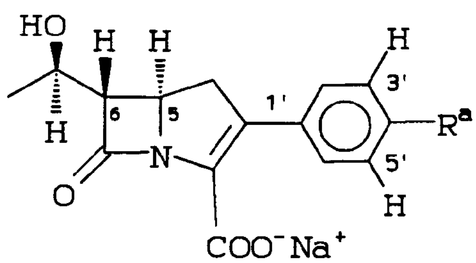

TABLE VI

| UV(nm) | | | |
|---|---|---|---|
| $R^a$ | $\lambda_{max}$ | $\varepsilon_{ext}$ | NMR ($D_20$) |
| F | ~298 | 6823 | 3.4-3.48(H6;dd;J=3&6Hz) 7-7.36 (AROMATIC H's) |
| Cl | ~303 | 6195 | 3.38-3.46(H6;dd;J=3&6Hz 7.2-7.35 (AROMATIC H's) |
| Br | ~304 | 8378 | 3.42-3.50(H6;dd;J=3&6Hz) 7.18-7.5 (AROMATIC H's) |
| I | ~307 | 9767 | 3.44-3.52(H6;dd;J+3&6Hz) 7.06-7.73 (AROMATIC H's) |

BIOLOGICAL DATA

The activity of the following compounds was measured against a strain of MRSA pathogen and reported as relative to the effectiveness of thienamycin, which is arbitrarily assigned an effectiveness of 1. Regarding this measure of activity, reference is made to R. Guthikonda, et al., J. Med. Chem., 30, 871 (1987).

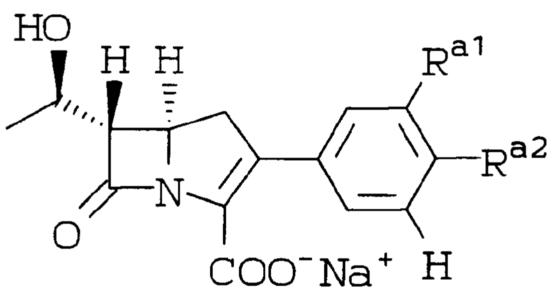

TABLE VII

| $R^{a1}$ | $R^{a2}$ | ANTI-INFECTIVE ACTIVITY MRSA |
|---|---|---|
| F | H | 2 |
| H | F | 1.1 |
| Cl | H | 8.1 |
| H | Cl | 1.3 |
| Br | H | 14 |
| H | Br | 1.6 |
| I | H | 21 |
| H | I | 1.8 |

## Claims

1. A compound of the formula:

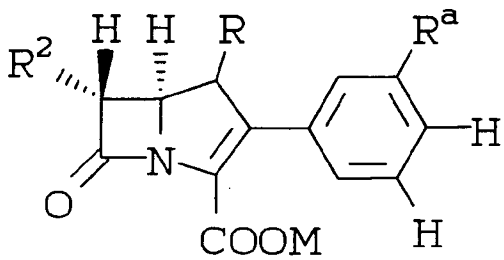

wherein R is H or $CH_3$, $R^2$ is (R)-$CH_3CH(OH)$- or (R)-$CH_3CH(F)$-, M is a pharmaceutically acceptable cation or ester and $R^a$ is I or Br.

2. The compound of claim 1 wherein $R^2$ is (R)-$CH_3CH(OH)$- and R is H.

3. The compound of claim 1 wherein M is a sodium or potassium cation.

4. A composition comprising a pharmaceutically acceptable carrier and from 0.1% to about 99% by weight of active material of claim 1.

5. A composition according to claim 4 which further comprises an inhibitorily effective amount of a DHP inhibitor.

6. A composition according to claim 5 wherein said DHP inhibitor is 7-(L-2-amino-2-carboxyethylthio)-2-(2,2-dimethylcyclopropanecarboxamide)-2-heptanoic acid.

7. The use of a compound of claim 1 for the manufacture of a medicament for treating bacterial infection in mammals.

8. The use according to claim 7 wherein the medicament manufactured further comprises an inhibitorily effective amount of a DHP inhibitor.

9. The use according to claim 8 wherein said DHP inhibitor is 7-(L-2-amino-2-carboxyethylthio)-2-(2,2-dimethylcyclopropanecarboxamide)-2-heptanoic acid.

European Patent Office

# EUROPEAN SEARCH REPORT

Application Number

EP 92 30 9350

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A<br><br>D | EP-A-0 010 316 (MERCK & CO., INC.)<br>* Page 82, table V, compound 4; Claims. *<br>& US-A-4 260 627<br>----- | 1-9 | C07D477/00<br>A61K31/40 |

TECHNICAL FIELDS SEARCHED (Int. Cl.5 )

C07D
A61K

The present search report has been drawn up for all claims

| Place of search | Date of completion of the search | Examiner |
|---|---|---|
| THE HAGUE | 05 JANUARY 1993 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)